(19) Europäisches Patentamt
European Patent Office
Office européen des brevets

(11) Veröffentlichungsnummer: **0 037 591**
**B1**

(12) # EUROPÄISCHE PATENTSCHRIFT

(45) Veröffentlichungstag der Patentschrift: **25.05.83**

(51) Int. Cl.³: **C 07 D 239/26**, A 01 N 43/54

(21) Anmeldenummer: **81104308.2**

(22) Anmeldetag: **07.09.78**

(60) Veröffentlichungsnummer der früheren Anmeldung nach Art. 76 EPÜ:

(54) **Phenoxy-pyrimidinyl-alkanole, Verfahren zu ihrer Herstellung sowie ihre Verwendung als Fungizide.**

(30) Priorität: **20.09.77 DE 2742173**

(43) Veröffentlichungstag der Anmeldung: **14.10.81 Patentblatt 81/41**

(45) Bekanntmachung des Hinweises auf die Patenterteilung: **25.05.83 Patentblatt 83/21**

(84) Benannte Vertragsstaaten: **BE CH DE FR GB NL**

(56) Entgegenhaltungen: **US-A-3 869 456**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Holmwood, Graham, Dr., Katernberger Strasse 184, D-5600 Wuppertal 1 (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen (DE)**
Erfinder: **Paul, Volker, Dr., Ahornstrasse 5, D-5650 Solingen (DE)**

**EP 0 037 591 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Phenoxy-pyrimidinyl-alkanole, Verfahren zu ihrer Herstellung sowie ihre Verwendung als Fungizide

Die Erfindung betrifft neue Phenoxy-pyrimidinyl-alkanole, ein Verfahren zu ihrer Herstellung sowie ihre Verwendung als Fungizide.

Es ist bereits bekanntgeworden, daß Trityl-1,2,4-triazole, wie Triphenyl-(1,2,4-triazol-1-yl)-methan, eine gute fungizide Wirksamkeit besitzen (vgl. DE-OS 1 795 249).

Deren Wirkung ist jedoch, insbesondere bei niedrigen Aufwandmengen und -konzentrationen, nicht immer ganz befriedigend. Weiterhin ist allgemein seit längerer Zeit bekannt, daß Zink-ethylen-1,2-bis-dithiocarbamidat ein gutes Mittel zur Bekämpfung von pilzlichen Pflanzenkrankheiten ist (vgl. Phytopathology, 33, 1113 [1963]). Jedoch ist dessen Einsatz als Saatgutbeizmittel nur beschränkt möglich, da es bei niedrigen Aufwandmengen und -konzentrationen wenig wirksam ist.

Ferner ist bereits bekannt, daß bestimmte 5-Pyrimidincarbinole zur Bekämpfung von phytopathogenen Pilzen geeignet sind (vgl. US-PS 3 869 456). Die Wirksamkeit dieser Stoffe läßt jedoch beim Einsatz geringer Aufwandmengen in manchen Fällen zu wünschen übrig.

Es wurden nun die neuen Phenoxy-pyrimidinyl-alkanole der allgemeinen Formel I

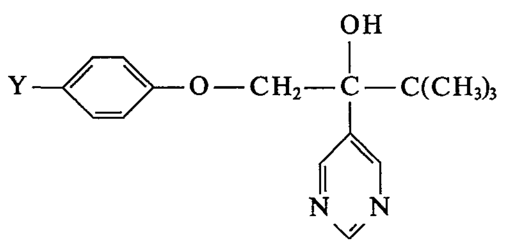

(I)

in welcher

Y für Fluor, Chlor oder Methyl steht,

gefunden. Sie weisen starke fungizide Eigenschaften auf.

Die Verbindungen der allgemeinen Formel I besitzen ein asymmetrisches Kohlenstoffatom; sie können deshalb in den beiden optischen Isomeren oder als Racemat vorliegen. Sämtliche Isomeren werden erfindungsgemäß beansprucht.

Weiterhin wurde gefunden, daß man die Phenoxy-pyrimidinyl-alkanole der allgemeinen Formel I erhält, wenn man Phenoxyketone der allgemeinen Formel II

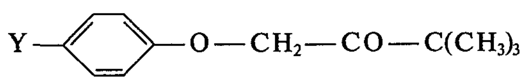

(II)

in welcher

Y die oben angegebene Bedeutung hat,

mit einem Pyrimidinyl-halogenid der allgemeinen Formel III

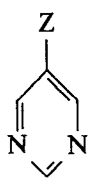

(III)

in welcher

Z für Halogen steht,

in Gegenwart eines Verdünnungsmittels und in Gegenwart einer alkalimetallorganischen Verbindung umsetzt.

Überraschenderweise zeigen die erfindungsgemäßen Phenoxy-pyrimidinyl-alkanole eine erheblich höhere fungizide Wirksamkeit, insbesondere gegen Mehltauarten, als die aus dem Stand der Technik bekannten Trityl-1,2,4-triazole, wie Triphenyl-(1,2,4-triazol-1-yl)-methan, und als Zink-ethylen-1,2-bis-dithiocarbamidat, welche bekannte Stoffe gleicher Wirkungsrichtung sind. Außerdem übertreffen die erfindungsgemäßen Stoffe auch die konstitutionell ähnlichsten 5-Pyrimidinyl-carbinole, die aus der US-PS 3 869 456 bekannt sind, bezüglich ihrer fungiziden Eigenschaften. So lassen sich die erfindungsgemäßen Stoffe besser zur Bekämpfung von phytopathogenen Pilzen verwenden als das 1-(4-Chlorphenyl)-1-(pyrimidin-5-yl)-5-phenoxy-pentan-1-ol, das 1-(3-Fluorphenyl)-1-(pyrimidin-5-yl)-2,2-dimethyl-propan-1-ol und das 1-(4-Methoxyphenyl)-1-(pyrimidin-5-yl)-2,2-dimethyl-propan-1-ol, welche in der US-PS 3 869 456 als Wirkstoffe mit fungiziden Eigenschaften offenbart werden. Die erfindungsgemäßen Wirkstoffe stellen somit eine Bereicherung der Technik dar.

Verwendet man 1-(4-Chlorphenoxy)-3,3-dimethyl-butan-2-on und 5-Brompyrimidin in Gegenwart

von n-Butyl-lithium als Ausgangsstoffe, so kann der Reaktionsablauf durch das folgende Formelschema wiedergegeben werden:

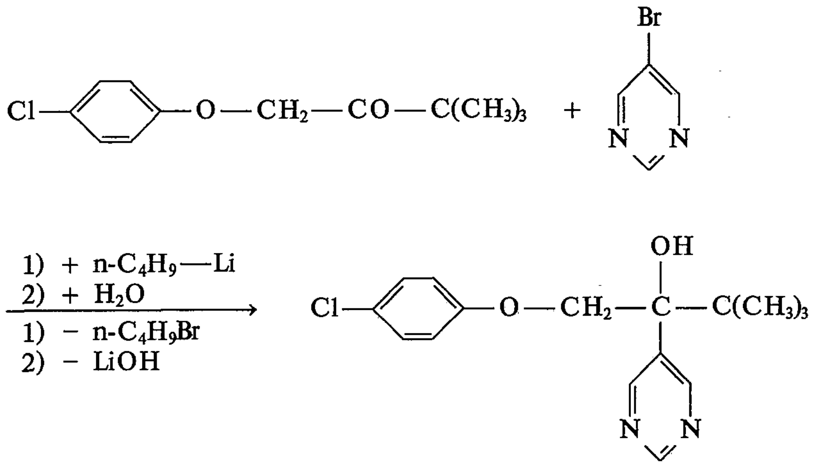


Die als Ausgangsstoffe zu verwendenden Phenoxyketone der allgemeinen Formel II sind bekannt oder lassen sich nach im Prinzip bekannten Verfahren in einfacher Weise herstellen (vgl. DE-OS 2 105 490 und 2 201 063). Man erhält sie zum Beispiel, indem man entsprechende Phenole mit entsprechenden Halogenketonen in Gegenwart eines Säurebinders und eines Verdünnungsmittels umsetzt.

Die außerdem als Ausgangsstoffe zu verwendenden Pyrimidinylhalogenide der allgemeinen Formel III, in denen Z vorzugsweise für Chlor oder Brom steht, sind allgemein bekannte Verbindungen der organischen Chemie.

Für die erfindungsgemäße Umsetzung kommen als Verdünnungsmittel vorzugsweise inerte organische Lösungsmittel in Frage. Hierzu gehören vorzugsweise solche, die einen tiefen Festpunkt besitzen, wie insbesondere Ether, wie Diethylether oder Tetrahydrofuran. Vorzugsweise arbeitet man mit Mischungen aus diesen beiden Ethern.

Als alkalimetallorganische Verbindungen werden bei der erfindungsgemäßen Umsetzung vorzugsweise Alkalimetallalkyle, wie insbesondere n-Butyl-lithium, eingesetzt. Es können aber auch Alkalimetall-aryle, wie Phenyllithium, Verwendung finden.

Die Reaktionstemperaturen können beim erfindungsgemäßen Verfahren in einem bestimmten Bereich variiert werden. Im allgemeinen arbeitet man zwischen −150 und −50°C, vorzugsweise zwischen −120 und −80°C.

Die erfindungsgemäße Umsetzung wird vorzugsweise unter Inertgas, wie insbesondere Stickstoff oder Argon, vorgenommen.

Bei der Durchführung des erfindungsgemäßen Verfahrens setzt man die Phenoxyketone der allgemeinen Formel II und die Halogenide der allgemeinen Formel III in etwa äquimolaren Mengen ein, Unter- oder Überschreitungen um bis zu ca. 20 Molprozent sind jedoch möglich. Die alkalimetallorganische Verbindung wird vorteilhaft im Überschuß von 5 bis 75 Molprozent, vorzugsweise von 10 bis 50 Molprozent, verwendet. Es kann dabei so verfahren werden, daß man zunächst die alkalimetallorganische Verbindung mit dem Halogenid der allgemeinen Formel III reagieren läßt und anschließend die Keto-Verbindung der allgemeinen Formel II zufügt; man kann aber auch die Keto-Verbindung und das Halogenid vorlegen und anschließend bei tiefer Temperatur (z. B. bei −100 bis −130°C) die alkalimetallorganische Verbindung zugeben.

Die Isolierung der Verbindung der allgemeinen Formel I erfolgt in der Weise, daß das bei der Reaktion zunächst gebildete Alkalialkanolat (z. B. Lithium-alkanolat) mit Wasser hydrolysiert wird. Die weitere Aufarbeitung erfolgt dann in üblicher Weise.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von echten Mehltaupilzen, beispielsweise zur Bekämpfung von Apfelmehltau (Podosphaera leucotricha) und Getreidemehltau sowie gegen andere Getreidekrankheiten verwendet werden; außerdem insbesondere zur Bekämpfung der Pilze Pyricularia und Pellicularia.

Besonders hervorzuheben ist die teilweise systemische Wirkung der Stoffe. So gelingt es, Pflanzen gegen Pilzbefall zu schützen, wenn man den Wirkstoff über den Boden und die Wurzel den oberirdischen Teilen der Pflanze zuführt.

Als Pflanzenschutzmittel können die erfindungsgemäßen Wirkstoffe zur Saatgut- oder Bodenbehandlung und zur Bekämpfung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z. B. Alkylaryl-polyglykol-ether, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozent, vorzugsweise zwischen 0,05 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Zur Bodenbehandlung sind Wirkstoffmengen von 1 bis 1000 g je cbm Boden, wie insbesondere 10 bis 200 g, erforderlich.

Die vielseitigen Verwendungsmöglichkeiten gehen aus den nachfolgenden Beispielen hervor.

## Beispiel A

### Sproßbehandlungs-Test/Getreidemehltau/protektiv
(blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Alkyl-aryl-polyglykolether auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 bis 22°C und einer Luftfeuchtigkeit von 80 bis 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Wirkstoffe, Wirkstoffkonzentrationen in der Spritzbrühe und Befallsgrade werden ermittelt. In diesem Test zeigen z. B. folgende Verbindungen eine sehr gute Wirkung, die derjenigen der aus dem Stand der Technik bekannten Verbindungen deutlich überlegen ist:

Verbindung gemäß Herstellungsbeispiel 1.

## Beispiel B

### Podosphaera-Test (Apfel)/Protektiv

| | |
|---|---|
| Lösungsmittel | 4,7 Gewichtsteile Aceton |
| Emulgator | 0,3 Gewichtsteile Alkyl-arylpolyglykolether |
| Wasser | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von ca. 70% gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. In diesem Test zeigen z. B. folgende Verbindungen eine sehr gute Wirkung, die derjenigen der aus dem Stande der Technik bekannten Verbindungen deutlich überlegen ist: Verbindung gemäß Herstellungsbeispiel 1.

## Beispiel C

### Myzelwachstums-Test

| | |
|---|---|
| 20 | Gewichtsteile Agar-Agar |
| 200 | Gewichtsteile Kartoffeldekokt |
| 5 | Gewichtsteile Malz |
| 15 | Gewichtsteile Dextrose |
| 5 | Gewichtsteile Pepton |
| 2 | Gewichtsteile Dinatriumhydrogenphosphat |
| 0,3 | Gewichtsteile Calciumnitrat |

Verhältnis von Lösungsmittelgemisch zum Nährboden:

| | |
|---|---|
| 2 | Gewichtsteile Lösungsmittelgemisch |
| 100 | Gewichtsteile Agarnährboden |

0,19 Gewichtsteile Dimethylformamid oder Aceton
0,01 Gewichtsteile Emulgator Alkylaryl-polyglykolether
1,80 Gewichtsteile Wasser
2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach der Wachstumsgeschwindigkeit der Pilze nach 4–6 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Wirkstoffe, Wirkstoffkonzentrationen und Resultate werden ermittelt. Dabei zeigen z. B. folgende Verbindungen eine sehr gute Wirkung, die derjenigen der aus dem Stand der Technik bekannten Verbindungen deutlich überlegen ist: Verbindung gemäß Herstellungsbeispiel 1.

## Herstellungsbeispiele

### Beispiel 1

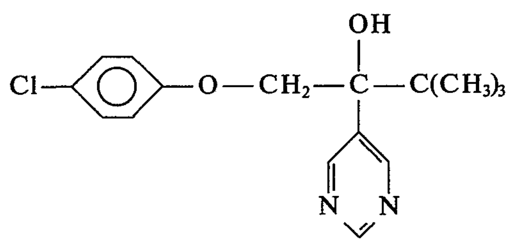

Eine Lösung von 22,65 g (0,1 Mol) 1-(4-Chlorphenoxy)-3,3-dimethyl-butan-2-on in 110 ml absolutem Tetrahydrofuran und 70 ml absolutem Ether wird unter trockener Stickstoffatmosphäre auf –120°C abgekühlt. Dazu tropft man eine Lösung von 15,9 g (0,1 Mol) 5-Brompyrimidin in 50 ml absolutem Tetrahydrofuran. Anschließend werden bei –120°C 50 ml einer 15%igen Lösung von n-Butyllithium in n-Hexan langsam zugetropft. Man läßt zunächst 2 Stunden bei einer Temperatur von ca. –110°C, danach über Nacht bei –78°C nachrühren. Das Reaktionsgemisch wird auf Raumtemperatur erwärmt, mit 100 ml 10%iger Ammoniumchlorid-Lösung und 200 ml Essigester versetzt und die wäßrige Phase abgetrennt. Die organische Phase wird nacheinander einmal mit 1 n-Salzsäure und zweimal mit gesättigter Natriumchloridlösung gewaschen, über Natriumsulfat getrocknet und eingeengt. Der Rückstand wird mit Ether aufgeschlämmt, der Feststoff abgesaugt und aus Acetonitril umkristallisiert. Man erhält 12,3 g (50% der Theorie bezogen auf n-Butyllithium) 1-(4-Chlorphenoxy)-3,3-dimethyl-2-(pyrimidin-5-yl)-butan-2-ol vom Schmelzpunkt 172–174°C.

In gleicher Weise werden die in den folgenden Beispielen aufgeführten Verbindungen hergestellt.

Beispiel 2

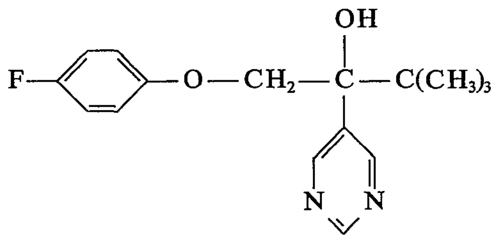

Schmelzpunkt: 163,5–164,5°C.

Beispiel 3

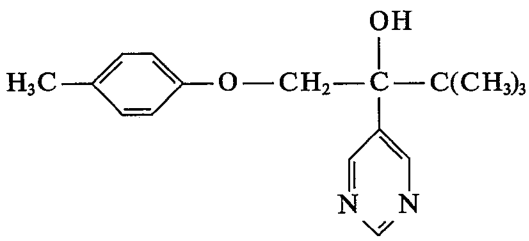

Schmelzpunkt: 152–153,5°C.

**Patentansprüche**

1. Phenoxy-pyrimidinyl-alkanole der allgemeinen Formel I

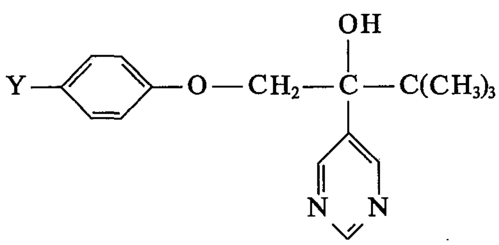

(I)

in welcher
Y für Fluor, Chlor oder Methyl steht.

2. Verfahren zur Herstellung von Phenoxy-pyrimidinyl-alkanolen der allgemeinen Formel I, dadurch gekennzeichnet, daß man Phenoxyketone der allgemeinen Formel II

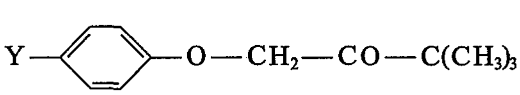

(II)

in welcher
Y die oben angegebene Bedeutung hat,
mit einem Pyrimidinyl-halogenid der allgemeinen Formel III

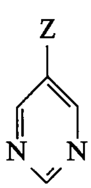

(III)

in welcher
Z für Halogen steht,
in Gegenwart eines Verdünnungsmittels und in Gegenwart einer alkalimetallorganischen Verbindung umsetzt.

3. Fungizide Mittel, gekennzeichnet durch einen Gehalt an mindestens einem Phenoxy-pyrimidinyl-alkanol der allgemeinen Formel I.

4. Verfahren zur Herstellung von fungiziden Mitteln, dadurch gekennzeichnet, daß man Phenoxy-pyrimidinyl-alkanole der allgemeinen Formel I mit Streckmitteln und/oder oberflächenaktiven Mitteln vermischt.

**Claims**

1. Phenoxy-pyrimidinyl-alkanols of the general formula I

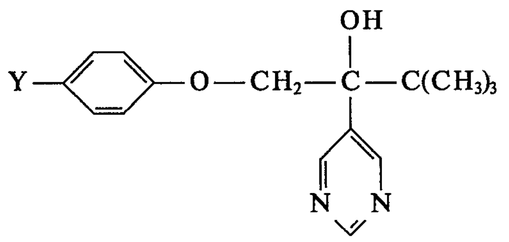

(I)

in which
Y represents fluorine, chlorine or methyl.

2. Process for the preparation of phenoxy-pyrimidinyl-alkanols of the general formula I, characterised in that phenoxyketones of the general formula II

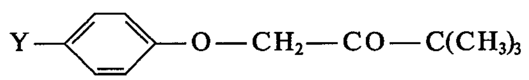

(II)

in which
Y has the meaning indicated above,
are reacted with a pyrimidinyl halide of the general formula III

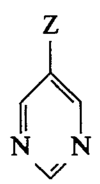

(III)

in which
Z represents halogen,
in the presence of a diluent and in the presence of an alkali metal-organic compound.

3. Fungicidal agents, characterised in that they contain at least one phenoxy-pyrimidinyl-alkanol of the general formula I.

4. Process for the preparation of fungicidal agents, characterised in that phenoxy-pyrimidinyl-alkanols of the general formula I are mixed with extenders and/or surface-active agents.

**Revendications**

1. Phénoxy-pyrimidinyl-alcanols de formule générale I:

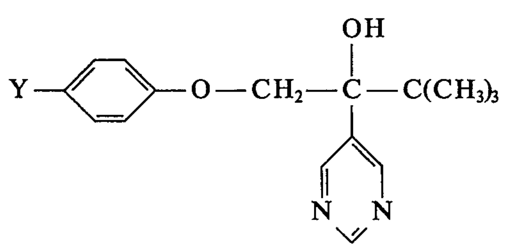

(I)

dans laquelle
Y représente un atome de fluor, un atome de chlore ou un groupe méthyle.

2. Procédé de préparation de phénoxy-pyrimidinyl-alcanols de formule générale I, caractérisé en ce qu'on fait réagir des phénoxy-cétones de formule générale II:

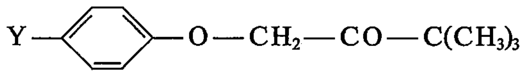

(II)

dans laquelle
Y a la signification indiquée ci-dessus,
avec un halogénure de pyrimidinyle de formule générale III:

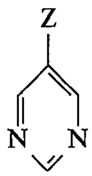

(III)

dans laquelle
Z représente un atome d'halogène,
en présence d'un diluant et également en présence d'un composé organométallique alcalin.

3. Agents fongicides, caractérisés en ce qu'ils contiennent au moins un phénoxy-pyrimidinyl-alcanol de formule générale I.

4. Procédé de préparation d'agents fongicides, caractérisé en ce qu'on mélange des phénoxy-pyrimidinyl-alcanols de formule générale I avec des diluants et/ou des agents tensio-actifs.